# EUROPEAN PATENT APPLICATION

(11) **EP 4 505 997 A1**
(43) Date of publication of application: **12.02.2025**
(21) Application number: 23795369.0
(22) Date of filing: 25.04.2023
(51) Int. Cl.: A61K 9/19, A61K 9/51, A61K 39/00, A61K 39/12, A61K 47/26, A61P 31/14, A61P 31/16, A61P 31/20, A61P 31/22, A61J 3/02

(54) **FREEZE-DRYING PROTECTIVE AGENT FOR NUCLEIC ACID-LIPID NANOPARTICLES, PREPARATION METHOD THEREFOR, AND APPLICATION THEREOF**

(30) Priority: 25.04.2022 CN 202210438888
(71) Applicant: Cansino Biologics Inc., Tianjin 300457 (CN); Cansino (Shanghai) Biological Research Co., Ltd., Shanghai 201203 (CN)
(72) Inventor: WANG, Haomeng, Tianjin 300457 (CN); LI, Mingyuan, Tianjin 300457 (CN); YAN, Zhihong, Tianjin 300457 (CN); JIA, Lin, Tianjin 300457 (CN); LIU, Jian, Tianjin 300457 (CN); MA, Wenlin, Tianjin 300457 (CN); QIU, Dongxu, Tianjin 300457 (CN); XIE, Yanbo, Tianjin 300457 (CN); YU, Xuefeng, Tianjin 300457 (CN); YU, Peng, Tianjin 300457 (CN); ZHU, Tao, Tianjin 300457 (CN)
(74) Representative: HGF
(86) International application number: PCT/CN2023/090473
(87) International publication number: WO 2023/207936

(57) **Abstract**

Based on the physicochemical principles of the freeze-drying process, the present invention performs systematic research on the freeze-drying conditions of nucleic acid-lipid nanoparticles, namely a freeze-drying process and a freeze-drying protective agent, optimizing and designing an efficient freeze-drying method suitable for the nucleic acid-lipid nanoparticles. According to the invention, the total duration of the nucleic acid-lipid nanoparticle freeze-drying process can be reduced to 8-18 hours, significantly reducing the energy consumption and the time cost of scaled-up product production; the freeze-dried nucleic acid-lipid nanoparticles rehydrate rapidly (within 10 seconds), and have a high total nucleic acid content, encapsulation efficiency and nucleic acid integrity; moreover, the cell transfection efficiency of a freeze-dried rehydrated preparation is not significantly different from that of a nucleic acid-lipid nanoparticle stock solution which has not been freeze-dried, and the in-vivo immune response is high and even exceeds that of a nucleic acid-lipid nanoparticle stock solution which has not been freeze-dried.

## Description

### TECHNICAL FIELD

The present disclosure belongs to the technical field of biological pharmaceuticals, and relates to a freeze-drying protective agent for nucleic acid-lipid nanoparticles, and a preparation method therefor and use thereof.

### BACKGROUND

In COVID-19 epidemic, mRNA vaccines, which have the advantages of safety, high immune response, and low production cost, were urgently approved for marketing in both the United States and the European Union. In recent years, studies have found that the transient protein expression induced by mRNA has great application value in various fields such as other infectious disease vaccines, cancer vaccines, cardiovascular diseases, protein replacement therapy, and genetic diseases, and can even realize the autonomous production of CAR-T effect through *in vivo* injection. However, unlike most of the vaccines, which are stored at 2-8°C, mRNA vaccines on the market have poor long-term stability, require cryopreservation (-20°C, even -70°C), and have an effective period of less than 6 months. In the face of hundreds of millions of doses of mRNA vaccines that need to be stored, transported and distributed around the world, stability becomes a bottleneck problem for their application.

First, mRNA strands are 1000 to 5000 bases in length, and it has been found in early studies that naked mRNAs will be rapidly hydrolyzed by ribonuclease (RNase), which is ubiquitous, adhering in large quantities both *in vivo* and *in vitro,* and requires special treatment to remove. In addition, only one change (bond break or base oxidation) in the long strand of mRNA will terminate the translation, so the integrity of mRNA molecules in mRNA vaccines is critical.

In order to improve the *in vivo* efficacy of mRNA in mRNA vaccines, LNP is used to encapsulate and deliver mRNA, which not only protects mRNA from transient destruction by RNase *in vivo,* but also breaks through the electrostatic repulsion barrier where both mRNA and cell membrane are negatively charged, and enhances the delivery to antigen presenting cells. However, mRNA-LNP injection has poor long-term stability, and in contrast, siRNA-LNP injection has good stability: Onpattro is very similar to LNP in mRNA-1273, but has an effective period of 3 years at 2-8°C. In addition, siRNA-LNP injection constructed by Suzuki et al. has good stability after being stored at 4°C for 1.5 years. siRNA has a small molecular weight and double-stranded structure, and mRNA has a single-stranded structure with a chain length more than 100 times that of siRNA, while the latter is more unstable and prone to hydrolysis. The above studies show that LNP cannot play a role in protecting the long-term stability of mRNA in injections.

LNP consists of four major components: neutral phospholipid, cholesterol, polyethylene glycol (PEG)-lipid, and ionizable cationic lipid. mRNA-LNP is found to be of a core-shell structure with a surface layer and an amorphous and isotropic core. Viger Gra et al. found that two types of cores are possible by using NMR spectroscopy: amorphous cores containing water pores surrounded by cationic lipids; or cores in which lipids can be uniformly dispersed, with a small water pocket arranged in the middle. Arteta et al. and Sebastiani et al. found that neutral phospholipids and PEG lipids as well as some ionizable cationic lipids and cholesterol are located on the surface of LNP, while ionizable cationic lipids, cholesterol, water, and mRNA are located in the core region. Studies have shown that LNP core contains 24% water and that mRNA is located in water column surrounded by cationic lipids.

Freeze drying, also known as sublimation drying, is a drying method in which an aqueous material is frozen below freezing point according to three-phase diagram of water, such that water is converted into ice, and subsequently, the ice is converted to vapor under high vacuum and removed. In recent years, freeze-dried products account for above half of biopharmaceuticals approved by FDA and EMA. In the field of nano-formulations, Ambisome^{®} and Vyxeos^{®} are both freeze-dried liposome formulations, both with a shelf life of up to 36 months. Dormitzer, Pfizer's viral vaccine research director, said that the company is interested in developing mRNA-LNP freeze-dried formulations. However, there are few studies on freeze-drying conditions of mRNA-LNPs, and although these mRNA-LNPs are all nano formulations, liposomes are closed vesicles formed by phospholipid bilayers, and the molecules entrapped in liposomes for freeze-drying are all small molecule drugs, mRNA-LNP is of a shell-core structure, the outer layer is only a phospholipid monomolecular layer, and mRNA entrapped in the core not only has a large molecular weight, but also has an extremely high requirement on integrity of mRNA molecules, it is necessary to keep the phospholipid layer and nanostructure of mRNA-LNP from being damaged in the high-intensity pressure change process of vacuum freeze-drying, ensure that the entrapment state of mRNA does not change, and restore the particle size distribution of a stock solution and the mRNA encapsulation efficiency after rehydration reconstruction, all of which are highly challenging. Zhao et al. prepared a novel freeze-dried formulation of lipid-like nanoparticles for mRNA delivery. Although the mRNA rehydrated formulation was not significantly different from the stock solution in *in vitro* cell viability, it did not have *in vivo* activity, and the reason for this is still unclear. Ai LX et al. separately entrapped S protein mRNA sequences of SARS-CoV-2 wild type, Delta strain, and Omicron strain by LNP, which could be stored at 4°C, 25°C, and 40°C for 18 days. Muramatsu et al. prepared an mRNA-LNP freeze-dried formulation, which could be stored at 4°C for 24 weeks, and had no significant difference in *in vivo* and *in vitro* biological activities. It can be seen that mRNA vaccines prepared under different freeze-drying conditions have great differences in *in vivo* biological activity, suggesting that different freeze-drying procedure curves and protective agents have great influences on the performance of the formulations, but the action mechanism and action rule thereof have not been reported in documents.

The freeze-drying process mainly includes two aspects: freeze-drying procedure curve and protective agent. The freeze-drying procedure curve is a curve of temperature, vacuum degree, energy, and the like in the freeze-drying process along with time, and generally includes three stages: pre-freezing, sublimation drying, and desorption drying. Cooling temperature, heating rate, maintenance time, and the like all have important influences on the appearance, moisture, particle size, and the like of freeze-dried powder. Freeze-drying protective agents, such as sucrose, trehalose, lactose, and mannitol, can stabilize the LNP structure by replacing hydrogen bonds between lipid molecules and water molecules during drying and dehydration, and act as excipients. The types, amounts, and adding sequence of freeze-drying protective agents all make the molecular action mechanism and microscopic space action different between the freeze-drying protective agents and mRNA-LNP, thereby having great influences on LNP membrane structure, mRNA encapsulation efficiency, mRNA integrity, and the like in freeze-dried powder. In conclusion, the influences of freeze-drying procedure curve and protective agent on the microstructure, key physicochemical properties, and biological activity of mRNA-LNP freeze-dried powder are in urgent need of in-depth investigation.

The key parameters of freeze-drying process in the prior art are usually applied in an "empirical" mode. Generally, freeze-drying process takes 30-100 h from pre-freezing to desorption drying, which results in energy consumption in one aspect, and greatly increases the time cost of scaled-up product production in another aspect. For example, the preferable freeze-drying procedure in the invention patent CN 110714015 B is "pre-freezing at a temperature of -50°C for 5 h, first freeze-drying at -40°C for 24 h, and second freeze-drying at 10°C for 17 h, with vacuum degree being 40 µbar in the freeze-drying process.", procedure which takes a total of 46 h. The freeze-drying procedure for mRNA-LNP freeze-dried formulation prepared by Muramatsu et al. takes a total of 84 h.

### SUMMARY

A first objective of the present disclosure is to improve the storage stability of nucleic acid-lipid nanoparticles, thereby reducing the storage condition to refrigeration (2-8°C) in one aspect, and prolonging the effective period in another aspect. Meanwhile, nucleic acid-lipid nanoparticles can be rehydrated rapidly.

A second objective of the present disclosure is to significantly shorten the total duration of a freeze-drying procedure, thereby increasing the efficiency, and reducing the energy and time costs of production.

A third objective of the present disclosure is to screen out specific types and amounts of freeze-drying protective agents for nucleic acid-lipid nanoparticles, which fully maintain the total amount, encapsulation efficiency, and integrity of nucleic acid in formulations after freeze-drying and rehydration, which have non-destructive *in vivo* and *in vitro* biological activity.

A fourth objective of the present disclosure is to break through the "empirical" development mode of freeze-drying process and establish a method for evaluating key physicochemical properties.

The term "lipid nanoparticle" of the present disclosure refers to a particle having at least one nanoscale dimension, which comprises at least one lipid.

The nucleic acid-lipid nanoparticle (LNP) described herein comprises components of nucleic acid, neutral lipid, protonatable cationic lipid, sterol lipid, and PEG-lipid.

The term "nucleic acid" of the present disclosure refers to a polymer containing at least two deoxyribonucleotides or ribonucleotides in either single- or double-stranded form, and includes DNA, RNA, and hybrids or derivatives thereof.

The term "lipid" of the present disclosure refers to a group of organic compounds, including, but not limited to, esters of fatty acids, which are generally characterized by being poorly soluble in water, but soluble in many organic solvents.

The term "protonatable cationic lipid" of the present disclosure refers to a lipid molecule that can be positively charged in certain environments, such as ALC-0315.

The term "neutral lipid" of the present disclosure refers to an uncharged non-phosphoglyceride lipid molecule.

The term "PEG-lipid (polyethylene glycol lipid)" of the present disclosure refers to a molecule comprising a lipid moiety and a polyethylene glycol moiety.

The term "vaccine" of the present disclosure refers to a composition suitable for application to an animal (including a human), which induces an immune response after administration with a strength sufficient to help prevent, improve, or cure clinical diseases caused by microbial infections as a minimum.

The term "delivery system" of the present disclosure refers to a formulation or composition that regulates the spatial, temporal and dose distribution of a biologically active ingredient in an organism.

Specifically, the nucleic acid-lipid nanoparticle, LNP, comprises, by mole percent, 25%-75% of protonatable cationic lipid, 5%-20% of neutral lipid, 0%-50% of sterol lipid, and 1%-5% of PEG lipid.

In order to achieve the above objectives of the present disclosure, the following technical solutions are adopted:
According to one aspect of the present disclosure, the present disclosure provides a freeze-drying protective agent for nucleic acid-lipid nanoparticles, which comprises the following components: sucrose and trehalose with a mass ratio of 5-15:5-11.

The freeze-drying protective agent for nucleic acid-lipid nanoparticles of the present disclosure comprises one or more of mannitol, glucose, or lactose.

According to one aspect of the present disclosure, in a formula of the freeze-drying protective agent of the present disclosure, the mass ratio of sucrose to trehalose to mannitol or glucose or lactose is 5-15:5-11:1-5.

According to one aspect of the present disclosure, the present disclosure provides a preparation method for the freeze-drying agent for nucleic acid-lipid nanoparticles, which comprises dissolving sucrose and trehalose in nuclease-free water according to corresponding proportion.

According to one aspect of the present disclosure, the present disclosure provides a preparation method for the freeze-drying agent for nucleic acid-lipid nanoparticles, which comprises dissolving sucrose, trehalose, and mannitol or glucose or lactose in nuclease-free water according to corresponding proportion.

According to one aspect of the present disclosure, the present disclosure provides use of the freeze-drying protective agent for nucleic acid-lipid nanoparticles in the manufacture of a medicament.

Preferably, the nucleic acid-lipid nanoparticles are added into a solution of the freeze-drying protective agent for freeze-drying.

Preferably, the freeze-drying comprises the following steps: pre-freezing, sublimation drying, and desorption drying.

Preferably, the pre-freezing is performed at a temperature of -80°C to -50°C for a period of 2-6 h.

Preferably, the sublimation drying is performed at a temperature of -60°C to 0°C, with an end temperature of -5°C to 0°C and vacuum pressure controlled to be ≤ 10 Pa; preferably, the sublimation drying is divided into 2-8 stages in a gradient heating mode, with each of the stages being in a heating gradient of 5-30°C and maintained for 0.5-10 h, and with vacuum pressure controlled to be ≤ 10 Pa.

Preferably, the desorption drying is performed at a temperature of 0-35°C, with an end temperature of 30-35°C, running time of 2-8 h, and vacuum pressure controlled to be ≤ 10 Pa.

Preferably, the nucleic acid-lipid nanoparticle comprises ssDNA, dsDNA, mRNA, IncRNA, siRNA, saRNA, shRNA, ASO (antisense oligonucleotide), plasmid, circRNA, circDNA, miRNA, CRISPR-Cas, ployI:C, SamRNA, and 5'-pppRNA.

Preferably, the medicament is a vaccine, more preferably, a vaccine for preventing cancer, viral infection, bacterial infection, and fungal infection; and more preferably, the virus is selected from: norovirus, Ebola virus, coronavirus, cytomegalovirus, Dengue virus, Zika virus, coxsackievirus, enterovirus, hepatitis virus, herpes simplex virus, human papilloma virus, influenza virus, Marburg virus, measles virus, poliovirus, rabies virus, rotavirus, and measles virus.

Preferably, the freeze-drying agent is added to the vaccine in an amount of 5%-20% (w/w%). Preferably, the medicament is an oral formulation, an intramuscular injectable formulation, an intravenous injectable formulation, or an inhalant formulation.

Preferably, the inhalation formulation is an aerosolized inhalant formulation or a dry powder inhalant formulation.

Compared with the prior art, the present disclosure has the following beneficial effects:
The present disclosure can shorten the total duration of nucleic acid-lipid nanoparticle freeze-drying process to 8-18 h, and significantly reduce the energy consumption and the time cost of scaled-up product production. Moreover, the freeze-dried nucleic acid-lipid nanoparticles prepared by the freeze-drying process of the present disclosure can be rehydrated rapidly (within 10 s), and has high total nucleic acid amount, encapsulation efficiency, and nucleic acid integrity. In addition, the formulation after freeze-drying and rehydration has cell transfection efficiency not significantly different from that of a stock solution of nucleic acid-lipid nanoparticles which has not been freeze-dried, and high *in vivo* immune response even exceeding that of the stock solution of nucleic acid-lipid nanoparticles which has not been freeze-dried. In addition, by reducing or even removing the water-containing microenvironment in the system through freeze-drying, nucleic acid hydrolysis catalyzed by nuclease can be inhibited, thereby significantly improving the long-term stability of the nucleic acid-lipid nanoparticles, prolonging the effective period, increasing the storage temperature to 2-8°C, and significantly reducing the cost and efficiency of storage and transportation. In addition, it is of great significance to convert the formulation phase state of nucleic acid-lipid nanoparticles from liquid to solid, which provides the possibility for the development of novel solid formulations.

### BRIEF DESCRIPTION OF THE DRAWINGS

To more clearly illustrate the specific embodiments of the present disclosure or the existing technical solutions in the prior art, the drawings needed in the description of the specific embodiments or the prior art are briefly described below. It is obvious that the drawings in the description below are only some embodiments of the present disclosure and those of ordinary skills in the art can acquire other drawings according to these drawings without creative efforts.
FIG. 1 is a diagram showing particle size distribution of the formulation after freeze-drying and rehydration in Example 1-1 after three measurements.
FIG. 2 is a scanning image of the sample obtained in Example 1-1 by SEM.
FIG. 3 is a scanning image of the sample obtained in Example 1-13 by SEM.
FIG. 4 is a scanning image of the sample obtained in Example 1-14 by SEM.
FIG. 5 is a scanning image of the sample obtained in Example 1-15 by SEM.
FIG. 6 is a scanning image of the sample obtained in Example 1-16 by SEM.
FIG. 7 is a scanning image of the sample obtained in Example 1-17 by SEM.
FIG. 8 is a scanning image of the sample obtained in Example 1-18 by SEM.
FIG. 9 is a scanning image of the sample obtained in Example 1-19 by SEM.
FIG. 10 is a scanning image of the sample obtained in Example 1-20 by SEM.
FIG. 11 is a scanning image of the sample obtained in Example 1-21 by SEM.
FIG. 12 is a scanning image of the sample obtained in Comparative Example 1-1 by SEM.
FIG. 13 is an image of microscopic morphology of the model sample by TEM.
FIG. 14 is an image of microscopic morphology of the sample obtained in Example 1-1 by TEM.
FIG. 15 is an image of microscopic morphology of the sample obtained in Example 1-4 by TEM.
FIG. 16 is an image of microscopic morphology of the sample obtained in Example 1-8 by TEM.
FIG. 17 is an image of microscopic morphology of the sample obtained in Example 1-12 by TEM.
FIG. 18 is an image of microscopic morphology of the sample obtained in Comparative Example 1-10 by TEM.
FIG. 19Ais a graph showing the mRNA integrity analysis test of the model sample.
FIG. 19B is a graph showing the mRNA integrity analysis test of the sample obtained in Example 2-1.
FIG. 19C is a graph showing the mRNA integrity analysis test of the sample obtained in Example 2-5.
FIG. 20 shows the protein expression levels in mRNA-LNP transfected cells.
FIG. 21 shows immune effect results 14 days after primary immunization.
FIG. 22 shows immune effect results 28 days after primary immunization (14 days after secondary immunization).

### DETAILED DESCRIPTION

The technical solutions of the present disclosure will be clearly and completely described below with reference to the examples of the present disclosure, and it is obvious that the described examples are a part of the examples of the present disclosure but not all of them. Based on the examples of the present disclosure, all other examples obtained by those of ordinary skills in the art without creative work shall fall within the protection scope of the present disclosure.

The technical solutions and beneficial effects of the present disclosure will be further described below with reference to preferred examples.

### Model sample:

mRNA encoding SARS-CoV-2 spike protein (S-mRNA, about 4000 bp) was used as model mRNA, and mRNAlipid nanoparticles (i.e., mRNA-LNPs) with no protective agent added and not subjected to freeze-drying was used as a model sample.

The preparation process was as follows: an mRNA stock solution was diluted to a concentration of 135 µg/mL with a sodium acetate buffer, and a lipid mixed solution was prepared according to a molar ratio of protonatable cationic lipid ALC-0315: DSPC: cholesterol: DMG-PEG 2000 of 49:10:39.5:1.5; after the encapsulation was completed on nano-drug manufacturing equipment, buffer exchange was performed by ultrafiltration, and the sample was collected.

### Liposome sample:

mRNA encoding SARS-CoV-2 spike protein (S-mRNA, about 4000 bp) was used as model mRNA, and mRNA liposome (i.e., mRNA-Lip) with no protective agent added and not subjected to freeze-drying was used as a liposome sample.

The preparation process was as follows: with DOTAP: DOPE at a molar ratio of 1:1 as a liposome membrane material, a cationic liposome was prepared by film dispersion, and subsequently mixed with mRNA for entrapment, with the concentration of mRNA adjusted to be consistent with that in the model sample.

### Example 1. Preparation of Freeze-Drying Protective Agents

Freeze-drying protective agents were formulated according to the formulas in Table 1, with nuclease-free water used as a solvent, and sterilized by filtration through 0.22 µm sterile filter membrane. The freeze-drying protective agent was added in an amount of 10% (w/w). Samples in Examples 1-1 to 1-12 and Comparative Examples 1-1 to 1-28 had protective agents uniformly mixed with the model sample lipid nanoparticles (i.e., mRNA-LNPs) and subpackaged into vials; Comparative Example 1-29 had the protective agent of Example 1-1 uniformly mixed with the liposome sample (i.e., nucleic acid-Lip), that is, the model sample was replaced with the liposome sample, and subpackaged into vials.

**Table 1. Formulas of freeze-drying protective agents**

| Freeze-drying protective agent /group | Component and proportion | | | | |
|---|---|---|---|---|---|
| | Sucrose | Trehalose | Mannitol | Glucose | Lactose |
| Example 1-1 | 5 | 5 | 5 | - | - |
| Example 1-2 | 5 | 5 | 1 | - | - |
| Example 1-3 | 5 | 11 | 1 | - | - |
| Example 1-4 | 5 | 11 | 5 | - | - |
| Example 1-5 | 15 | 5 | 1 | - | - |
| Example 1-6 | 15 | 5 | 5 | - | - |
| Example 1-7 | 15 | 11 | 1 | - | - |
| Example 1-8 | 15 | 11 | 5 | - | - |
| Example 1-9 | 5 | 5 | - | - | - |
| Example 1-10 | 5 | 11 | - | - | - |
| Example 1-11 | 15 | 5 | - | - | - |
| Example 1-12 | 15 | 11 | - | - | - |
| Example 1-13 | 15 | 11 | 2.5 | 2.5 | - |
| Example 1-14 | 15 | 11 | 2.5 | - | 2.5 |
| Example 1-15 | 15 | 11 | 2.5 | 2.5 | 2.5 |
| Example 1-16 | 7.5 | 11 | 5 | 7.5 | - |
| Example 1-17 | 7.5 | 11 | 5 | - | 7.5 |
| Example 1-18 | 7.5 | 11 | 5 | 7.5 | 7.5 |
| Example 1-19 | 15 | 5.5 | 5 | 5.5 | |
| Example 1-20 | 15 | 5.5 | 5 | | 5.5 |
| Example 1-21 | 15 | 5.5 | 5 | 5.5 | 5.5 |
| Comparative Example 1-1 | 100% | - | - | - | - |
| Comparative Example 1-2 | - | 100% | - | - | - |
| Comparative Example 1-3 | - | - | 100% | - | - |
| Comparative Example 1-4 | 5 | 4 | 5 | - | - |
| Comparative Example 1-5 | 5 | 12 | 1 | - | - |
| Comparative Example 1-6 | 5 | 12 | 5 | - | - |
| Comparative Example 1-7 | 15 | 4 | 1 | - | - |
| Comparative Example 1-8 | 15 | 4 | 5 | - | - |
| Comparative Example 1-9 | 15 | 12 | 1 | - | - |
| Comparative Example 1-10 | 5 | 5 | 6 | - | - |
| Comparative Example 1-11 | 5 | 11 | 0.5 | - | - |
| Comparative Example 1-12 | 5 | 11 | 6 | - | - |
| Comparative Example 1-13 | 15 | 5 | 0.5 | - | - |
| Comparative Example 1-14 | 15 | 5 | 6 | - | - |
| Comparative Example 1-15 | 15 | 11 | 0.5 | - | - |
| Comparative Example 1-16 | 4 | 5 | 5 | - | - |
| Comparative Example 1-17 | 4 | 11 | 1 | - | - |
| Comparative Example 1-18 | 4 | 11 | 5 | - | - |
| Comparative Example 1-19 | 16 | 5 | 1 | - | - |
| Comparative Example 1-20 | 16 | 5 | 5 | - | - |
| Comparative Example 1-21 | 16 | 11 | 1 | - | - |
| Comparative Example 1-22 | 15 | 11 | - | 5 | - |
| Comparative Example 1-23 | 15 | 11 | - | - | 5 |
| Comparative Example 1-24 | - | 11 | 5 | 15 | - |
| Comparative Example 1-25 | - | 11 | 5 | - | 15 |
| Comparative Example 1-26 | 15 | - | 5 | 11 | - |
| Comparative Example 1-28 | 15 | - | 5 | - | 11 |
| Comparative Example 1-29 | 5 | 5 | 5 | | |

500 µL of each of the samples of Examples 1-1 to 1-21 and Comparative Examples 1-1 to 1-29 was added to 3 mL vial, and freeze-dried. Freeze-drying procedure: pre-freezing stage: -60°C for 4 h; sublimation drying stage: -45°C for 2 h, -25°C for 2 h, -5°C for 2 h, with vacuum pressure controlled to be ≤ 10 Pa in the whole process; and desorption drying stage: 30°C for 2 h, with vacuum pressure controlled to be ≤ 10 Pa in the whole process.

Determination of particle size, potential, and rehydration time: the model sample, and samples of Examples 1-1 to 1-21 and Comparative Examples 1-1 to 1-29 were separately taken (0.5 mL of nuclease-free water was added to each of the freeze-dried samples of Examples and Comparative Examples for rehydration, and the time required for rehydration was recorded), and 1 mL of nuclease-free water was supplemented. The particle size, polydispersity coefficient, and Zeta potential were measured at 25 ± 1°C in a Malvern particle sizer, in parallel for three times, and the average value was taken, as shown in Table 2.

**Table 2. Particle size and potential**

| Sample name | Average particle size of three measurements | PDI | Zeta potential | Rehydration time s |
|---|---|---|---|---|
| Model sample | 99.02 | 0.124 | -4.31 | - |
| Liposome sample | 102.31 | 0.135 | +18.6 | - |
| Example 1-1 | 114.5 | 0.134 | -3.13 | 5 |
| Example 1-2 | 116.6 | 0.162 | -6.45 | 6 |
| Example 1-3 | 115.1 | 0.164 | -6.71 | 4 |
| Example 1-4 | 117.5 | 0.142 | -5.06 | 5 |
| Example 1-5 | 116.9 | 0.148 | -4.99 | 6 |
| Example 1-6 | 110.7 | 0.166 | -7.12 | 4 |
| Example 1-7 | 115.8 | 0.159 | -6.45 | 6 |
| Example 1-8 | 111.3 | 0.139 | -4.83 | 4 |
| Example 1-9 | 121.4 | 0.168 | -6.24 | 7 |
| Example 1-10 | 120.9 | 0.173 | -5.87 | 7 |
| Example 1-11 | 123.3 | 0.179 | -4.93 | 7 |
| Example 1-12 | 122.1 | 0.169 | -5.42 | 7 |
| Example 1-13 | 113.9 | 0.155 | -5.35 | 8 |
| Example 1-14 | 113.9 | 0.155 | -5.35 | 8 |
| Example 1-15 | 119.2 | 0.166 | -7.34 | 7 |
| Example 1-16 | 115.4 | 0.139 | -6.79 | 9 |
| Example 1-17 | 112.8 | 0.166 | -5.23 | 6 |
| Example 1-18 | 120.3 | 0.178 | -4.63 | 8 |
| Example 1-19 | 116.7 | 0.161 | -3.91 | 10 |
| Example 1-20 | 121.8 | 0.148 | -4.58 | 7 |
| Example 1-21 | 122.1 | 0.196 | -4.77 | 10 |
| Comparative Example 1-1 | 118.5 | 0.168 | -6.84 | 9 |
| Comparative Example 1-2 | 186.5 | 0.173 | -3.78 | 12 |
| Comparative Example 1-3 | 162.1 | 0.168 | -4.51 | 10 |
| Comparative Example 1-4 | 272.5 | 0.256 | -3.77 | 13 |
| Comparative Example 1-5 | 186.8 | 0.197 | -5.23 | 11 |
| Comparative Example 1-6 | 415.5 | 0.475 | -5.74 | 35 |
| Comparative Example 1-7 | 347.7 | 0.392 | -6.22 | 36 |
| Comparative Example 1-8 | 180.5 | 0.185 | -7.04 | 12 |
| Comparative Example 1-9 | 171.4 | 0.209 | -6.12 | 14 |
| Comparative Example 1-10 | 202.3 | 0.223 | -5.58 | 18 |
| Comparative Example 1-11 | 521.21 (foaming, no dried powder was obtained) | 0.345 | -3.23 | 84 |
| Comparative Example 1-12 | 267.3 | 0.302 | -4.35 | 27 |
| Comparative Example 1-13 | 138.0 | 0.191 | -3.98 | 15 |
| Comparative Example 1-14 | 403.42 (solute loss) | 0.289 | -6.74 | 34 |
| Comparative Example 1-15 | 182.9 | 0.231 | -4.67 | 15 |
| Comparative Example 1-16 | 374.8 | 0.316 | -6.34 | 47 |
| Comparative Example 1-17 | 318.8 | 0.372 | -7.02 | 35 |
| Comparative Example 1-18 | 275.2 | 0.289 | -5.94 | 15 |
| Comparative Example 1-19 | 203.6 | 0.183 | -4.23 | 16 |
| Comparative Example 1-20 | 192.2 | 0.221 | -5.31 | 19 |
| Comparative Example 1-21 | 321.9 | 0.373 | -6.33 | 73 |
| Comparative Example 1-22 | 140.8 | 0.194 | -5.29 | 12 |
| Comparative Example 1-23 | 153.2 | 0.217 | -4.31 | 17 |
| Comparative Example 1-24 | 237.9 | 0.281 | -5.33 | 13 |
| Comparative Example 1-25 | 144.8 | 0.199 | -3.94 | 19 |
| Comparative Example 1-26 | 139.3 | 0.189 | -4.18 | 11 |
| Comparative Example 1-28 | 150.6 | 0.221 | -4.73 | 19 |
| Comparative Example 1-29 | 178.3 | 0.242 | +16.9 | 8 |

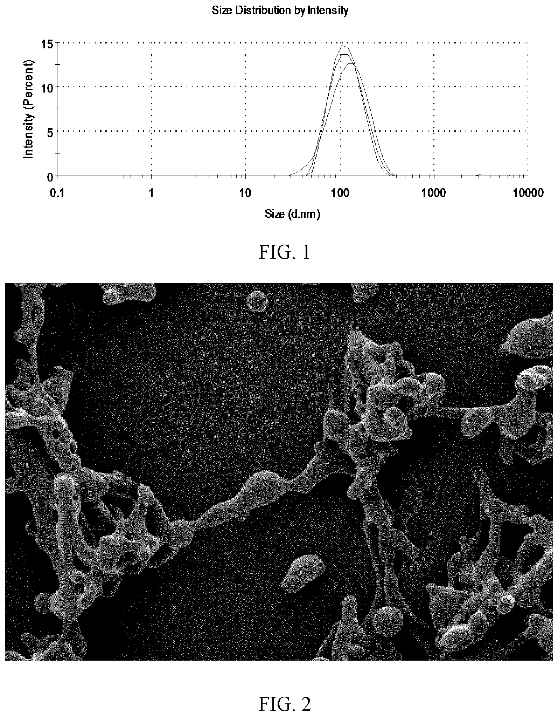

It could be seen that Examples 1-1 to 1-12 showed an increase of < 25 nm in the particle size compared with the model sample. The particle size distribution of the freeze-dried powder obtained by the freeze-drying process of Example 1-1 after rehydration is shown in FIG. 1. Comparative Examples 1-1 to 1-28 showed an increase of about 60-320 nm in the particle size compared with the model sample, suggesting that the nanostructure of nucleic acid-lipid nanoparticles has been damaged. It can be seen that the combination of sucrose and trehalose as a freeze-drying protective agent has a protective effect exceeding that of the two protective agents alone, with the combination of sucrose, alginate, and mannitol being the most effective. There are various stress damages in freeze-drying process, such as low-temperature stress, freezing stress, and drying stress, in which the freezing stress can be divided into four situations: the formation of dendritic ice crystals, the increase in ion concentration, the change in pH value, and phase separation. When sucrose, trehalose, and mannitol are mixed at a proper ratio and added in a proper amount to mRNA-LNPs for freeze-drying, a more compact wrapping layer is formed by the three due to molecular structure characteristics dominated by van der Waals attraction among them, which completely wraps the mRNA-LNPs to form a complete ginger-shaped wrapping scaffold in the whole system. In one aspect, the powder cake has strong rigidity, is not easy to disintegrate, can resist various stress damages, and has good stability; in another aspect, it has high porosity, which is beneficial to rapid evaporation of water in freeze-drying process, and the effect of rapid rehydration can be achieved due to the large specific surface area wetted by water molecules in rehydration process. Although the protective agent of Example 1-1 was identical to Comparative Example 1-29, Comparative Example 1-29 showed an increase of greater than 75 nm in the particle size compared with the liposome sample, indicating that the protective agent system has nucleic acid-LNP specificity, that is, it is suitable for nucleic acid-LNP formulations but not for nucleic acid-Lip.

### Example 2. Morphologic Observation by Scanning Electron Microscope (SEM)

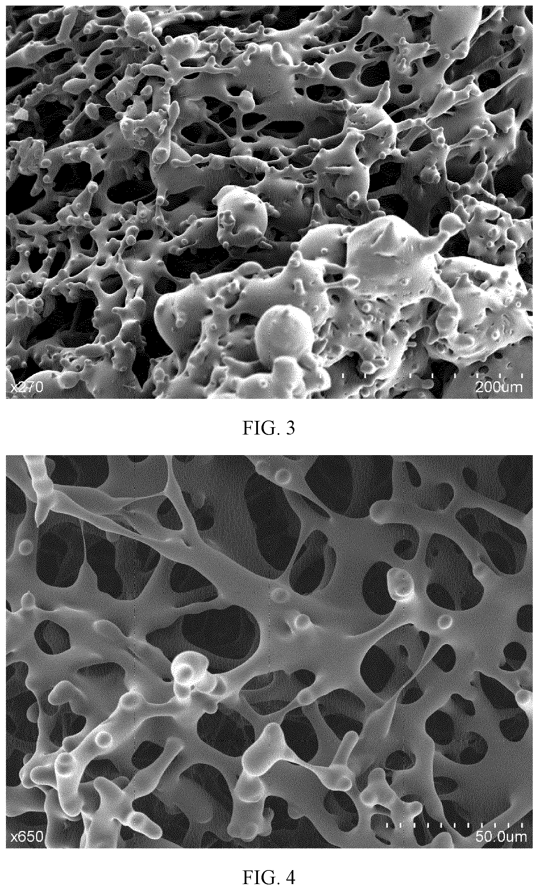

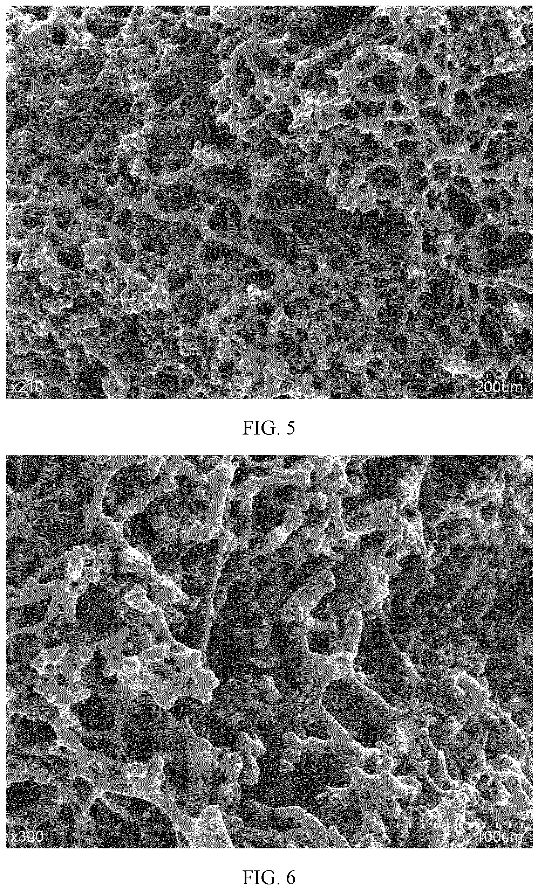

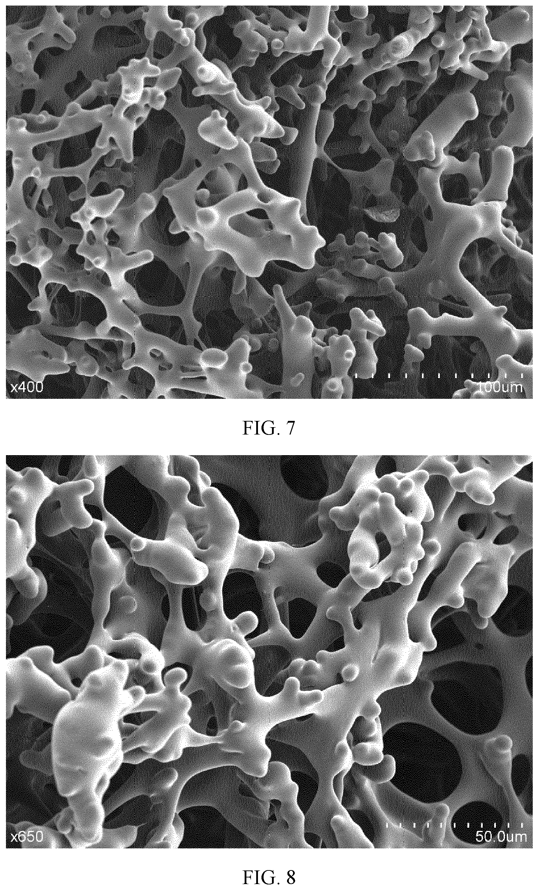

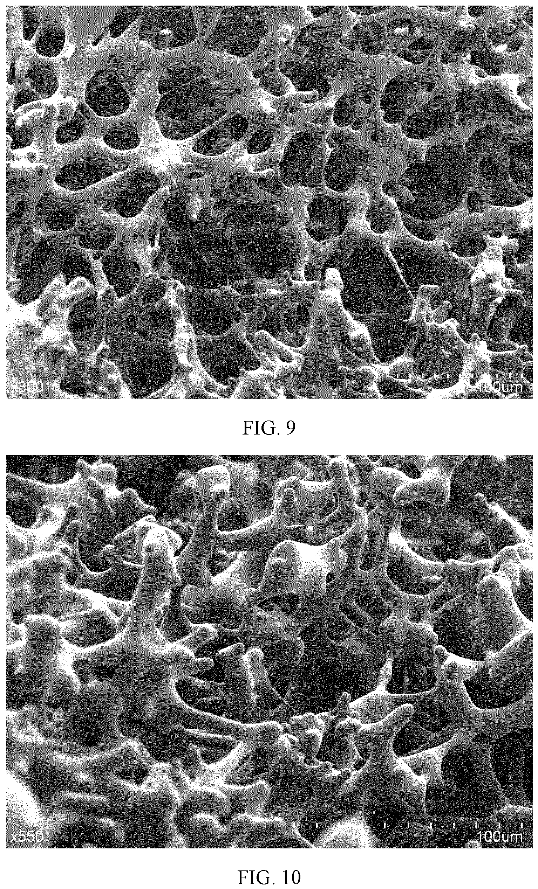

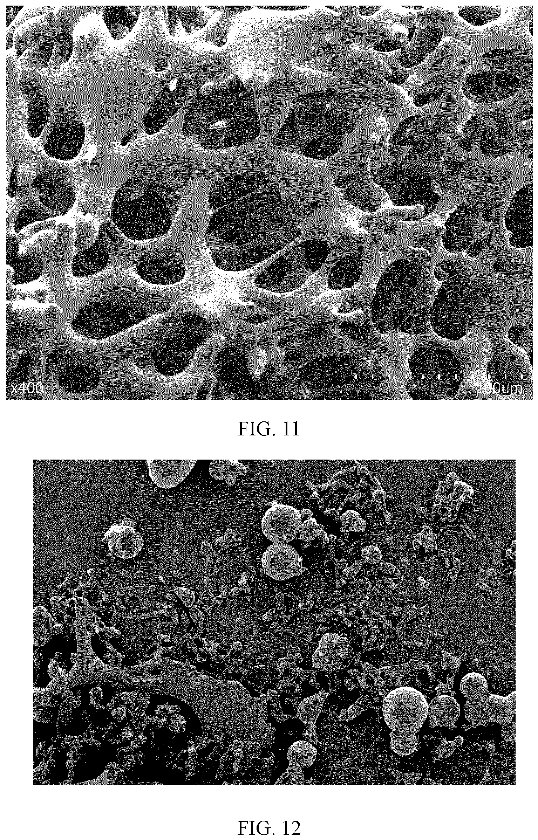

Operation steps of scanning electron microscope (SEM): a small amount of freeze-dried powder sample of each of Examples 1-1, 1-13, 1-14, 1-15, 1-16, 1-17, 1-18, 1-19, 1-20, and 1-21 and Comparative Example 1-1 was placed on conductive adhesive, and then scanned and imaged by SEM. The results are shown in FIGs. 2-12.

It could be seen that Examples were of a "ginger-shaped" wrapping scaffold microstructure, while in Comparative Example, a scaffold structure was not formed between the LNP and the protective agent, indicating that the protective agent could only play a role in dispersing and filling and reducing stress damage.

### Example 3. Morphologic Observation by Transmission Electron Microscopy (TEM)

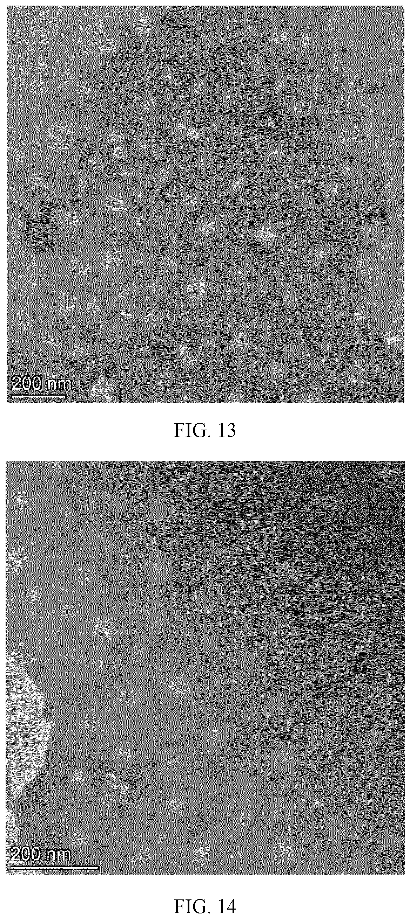

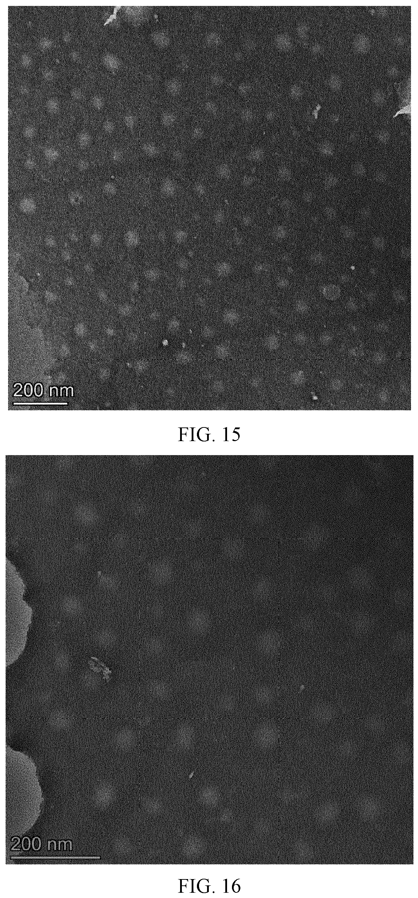

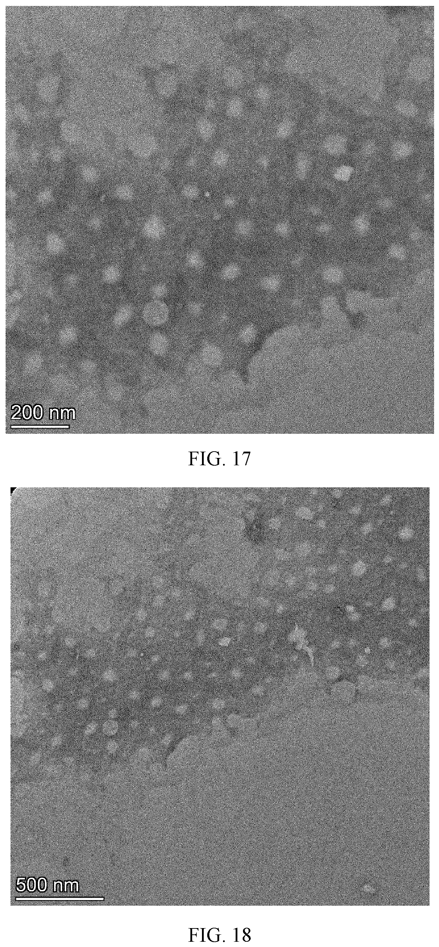

The model sample and Example 1-1, Example 1-4, Example 1-8, Example 1-12 and Comparative Example 1-10 were diluted with a proper amount of nuclease-free water. The diluted samples were each added dropwise on a copper mesh, the redundant sample was removed from the edge of the copper mesh with filter paper, then 2% phosphotungstic acid was added dropwise for dyeing for 5 s, and the redundant phosphotungstic acid was removed from the edge of the copper mesh with filter paper. The copper mesh was scanned and imaged by using a transmission electron microscope. The results are shown in FIGs. 13-18. It could be seen that, compared to Comparative Example, Examples had similar particle size distribution and microscopic morphology to the model sample.

### Example 4. Freeze-drying Curve Experiment

500 µL of the sample of Example 1-8 was added to 3 mL vial, and freeze-dried. Freeze drying procedure: pre-freezing stage: -60°C for 4 h; desorption drying stage: 30°C for 2 h, with vacuum pressure controlled to be ≤ 10 Pa in the whole process; and sublimation drying stage: performed according to the procedures of Table 3, Examples 2-1 to 2-6 and Comparative Examples 2-1 to 2-6 were obtained.

**Table 3. Sublimation drying procedure**

| Experimental group | Sublimation drying procedure | |
|---|---|---|
| | | |
| Example 2-1 | -45°C 2h, -25°C 4h, -5°C 2h | The vacuum pressure was controlled to be ≤ 10 Pa in the whole process. |
| Example 2-2 | -50°C 2h, -35°C 4h, -10°C 2h. | |
| Example 2-3 | -45°C 4h, -10°C 4h | |
| Example 2-4 | -50°C 4h, -25°C 4h | |
| Example 2-5 | -45°C 2h, -35°C 2h, -25°C 2h, -10°C 1h, -5°C 1h | |
| Example 2-6 | -50°C 2h, -35°C 1h, -30°C 1h, -25°C 1h, -20°C 1h, -10°C 1h, -5°C 1h | |
| Comparative Example 2-1 | -50°C 8h | |
| Comparative Example 2-2 | -45°C 8h | |
| Comparative Example 2-3 | -35°C 8h | |
| Comparative Example 2-4 | -25°C 8h | |
| Comparative Example 2-5 | -10°C 8h | |
| Comparative Example 2-6 | -5°C 8h | |

### Determination of mRNA content, encapsulation efficiency, and integrity and stability characterization

Storage conditions for stability: 2-8°C for 1 month. The model sample, Examples 2-1 to 2-6, and Comparative Examples 2-1 to 2-6 were determined for total amount of mRNA, free mRNA concentration, encapsulation efficiency, and mRNA integrity at 0 point and month 1. The results are shown in Table 4.

The RiboGreen RNA reagent bound to free mRNA for fluorescent color development, and the amount of free mRNA was determined by using a fluorescence analyzer; meanwhile, the total amount of mRNA was determined by membrane rupture with Triton X-100, and subsequently: encapsulation efficiency (EE%) = (total amount of mRNA - free amount of mRNA)/ total amount of mRNA × 100%

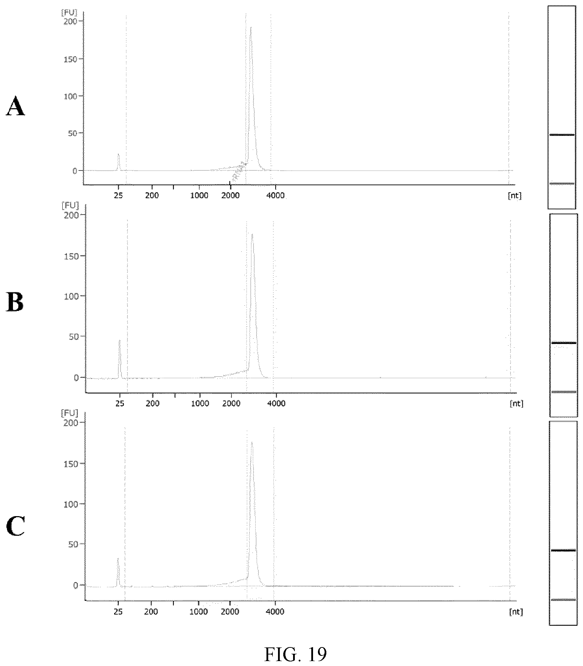

Determination of mRNA integrity: the mRNA-LNP sample was first diluted and denatured at 70°C, Dye-Gel glue was prepared and injected into a glue pressing device, and subsequently, mRNA marker, ladder, and sample were sequentially added. The mRNA integrity profiles of the samples were efficiently determined by using an Agilent 2100 bioanalysis system according to the principle of gel electrophoresis. The mRNA integrity profiles of the model sample, Example 2-1, and Example 2-5 at 0 point are shown in FIG. 19 (A/B/C). The experimental results showed that the intermediate-temperature and multi-step heating mode in the freeze-drying process is more favorable for protecting the nanostructure of nucleic acid-lipid nanoparticles, increasing the encapsulation efficiency and integrity of mRNA, and improving the stability of freeze-dried formulations.

**Table 4. mRNA concentration encapsulation efficiency, and integrity**

| Sample name | Time point | Total mRNA concentration µg/mL | Free mRNA concentration µg/mL | EE (%) | mRNA integrity (%) |
|---|---|---|---|---|---|
| Model sample | 0 point | 90.24 | 6.02 | 93.33 | 89.4 |
| | Month 1 | 84.23 | 11.62 | 86.21 | 80.2 |
| Example 2-1 | 0 point | 90.17 | 6.81 | 92.45 | 87.5 |
| | Month 1 | 90.15 | 6.84 | 92.41 | 87.4 |
| Example 2-2 | 0 point | 90.01 | 8.88 | 90.13 | 85.3 |
| | Month 1 | 89.92 | 8.92 | 90.08 | 84.9 |
| Example 2-3 | 0 point | 90.12 | 7.55 | 91.62 | 86.6 |
| | Month 1 | 90.09 | 7.68 | 91.48 | 86.4 |
| Example 2-4 | 0 point | 90.29 | 6.90 | 92.36 | 87.2 |
| | Month 1 | 89.33 | 7.03 | 92.13 | 87.0 |
| Example 2-5 | 0 point | 90.51 | 7.49 | 91.72 | 86.5 |
| | Month 1 | 90.02 | 7.90 | 91.22 | 86.3 |
| Example 2-6 | 0 point | 90.12 | 7.35 | 91.84 | 87.0 |
| | Month 1 | 90.00 | 7.79 | 91.35 | 86.8 |
| Comparative Example 2-1 | 0 point | 88.78 | 31.67 | 64.33 | 65.8 |
| | Month 1 | 86.23 | 43.23 | 49.87 | 56.2 |
| Comparative Example 2-2 | 0 point | 86.47 | 31.43 | 63.65 | 58.6 |
| | Month 1 | 83.66 | 35.25 | 57.86 | 54.3 |
| Comparative Example 2-3 | 0 point | 87.05 | 42.23 | 51.49 | 35.7 |
| | Month 1 | 84.53 | 45.94 | 45.65 | 28.9 |
| Comparative Example 2-4 | 0 point | 86.47 | 31.43 | 63.65 | 58.6 |
| | Month 1 | 85.43 | 34.40 | 59.73 | 57.3 |
| Comparative Example 2-5 | 0 point | 87.77 | 32.95 | 62.46 | 58.1 |
| | Month 1 | 83.93 | 36.69 | 56.28 | 55.9 |
| Comparative Example 2-6 | 0 point | 85.11 | 33.91 | 60.16 | 57.9 |
| | Month 1 | 84.94 | 36.36 | 57.19 | 52.8 |

### Example 5. Cell Transfection Effect - Western Blot Assay

HEK293 cells were used as model cells for protein expression *in vitro.* The cells were plated in a 6-well plate with a cell density of 1 × 10⁶ cells/mL and cultured for 24 h. Subsequently, Example 1-1, Example 1-4, Example 1-8, and Example 1-12 were separately reconstituted with nuclease-free water, the model sample was taken, and they were sequentially added to the wells at an amount of 20 µL. After mixing uniformly, the transfection was performed for 4 h, and the medium (containing serum) was changed. After culturing for 24 h in an incubator, the cells were digested with PBS, and then transferred to a centrifuge tube, and centrifuged (2500 r/min, 5 min).

The cells were collected, added with 1 mL of PBS, uniformly mixed by pipetting, transferred to 1.5 mL EP tube, and centrifuged. The cells in the lower layer were taken. 200 µL of protein lysis buffer (adding 1 µL of protease inhibitor per 100 µL of CHAPS) was added to each of the samples for lysis on ice for 1 h. The lysate was centrifuged in a centrifuge at 4°C (13500 r/min, 20 min), and the supernatant was collected. The sample was determined for protein expression level by western blot.

First, leakage detection was performed on an electrophoresis device (detecting the leakage with water, filling up the electrophoresis device with water, and if the liquid level does not drop within 5 min, the electrophoresis device can be used), and after the leakage detection was completed, the water was cleaned up; the prepared 12% separation gel (volume fraction) was added into the electrophoresis device, followed by absolute ethanol (for pressing bubbles), the electrophoresis device was left to stand for 30 min. After the separation gel solidified, the absolute ethanol was poured off, and the redundant liquid was absorbed with filter paper. A stacking gel was added, a comb was rapidly inserted, and the electrophoresis device was left to stand for 30 min for solidifying the stacking gel. The device was placed in an electrophoresis tank, an electrophoresis solution (1× SDS-PAGE buffer) was added to immerse platinum wire, the comb was pulled out, and the protein expression samples of Example 1-1, Example 1-4, Example 1-8, and Example 1-12, and the model samples were sequentially added. Electrophoresis (100 V, 300 mA) was performed, during which the positions of bromophenol blue and marker were observed, and when they reached the bottom of the gel, the electrophoresis was stopped. The gel after electrophoresis was taken out and separated according to the boundary between the stacking gel and the separation gel, the stacking gel was removed, the separation gel was retained, and subsequently, the separation gel and the filter paper were soaked in 1× blotting buffer, and PVDF membrane was soaked in anhydrous methanol to be activated. 3 layers of filter paper, 1 layer of PVDF membrane, 1 layer of separation gel, and 3 layers of filter paper were sequentially placed on a membrane transfer instrument, bubbles were expelled, power supply was turned, and transfer was performed for 1 h 40 min at 15 V (gradient boosting from 5 V to 15 V), 300 mA. The PVDF membrane (with protein) was soaked in 1× PBST containing 5% skimmed milk powder, blocked at room temperature for 2 h, and subsequently washed with 1× PBST to remove excessive skimmed milk powder. The membrane was placed in 15 mL centrifuge tube containing primary antibody (diluted with 5% skimmed milk powder according to instructions), incubated at 4°C overnight, and washed with 1× PBST to remove excess primary antibody. The PVDF membrane with the primary antibody blocked was placed into 15 mL centrifuge tube containing secondary antibody (diluted with 5% skim milk powder according to instructions), incubated at room temperature for 1 h (be careful to avoid light), and washed to remove excess secondary antibody, and subsequently, the membrane was detected and analyzed by an Odyssey infrared laser imaging system.

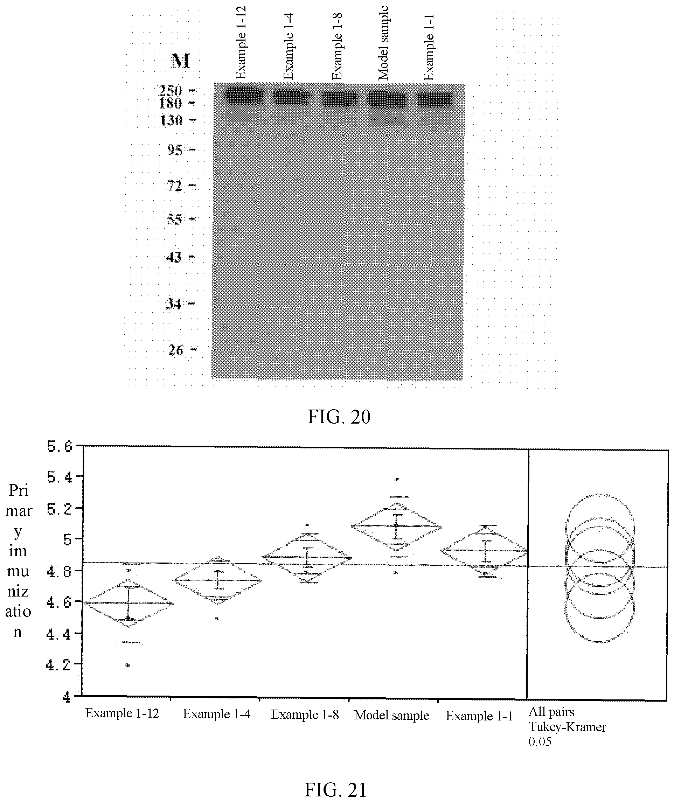

The experimental results are shown in FIG. 20. It could be seen that there was no significant difference in S protein expression level between the rehydrated formulations of Example 1-1, Example 1-4, Example 1-8, and Example 1-12 and the model sample, indicating that there was no significant difference in cell transfection efficiency among all groups, and the freeze-drying process did not reduce the biological activity of mRNA-LNP at the cellular level.

### Example 6. In Vivo Immune Effect

BALB/c mice were taken. Comparative studies of rehydrated formulations of Example 1-1, Example 1-4, Example 1-8, and Example 1-12 with the model sample were performed. The mice were randomly divided into 5 groups, which were injected intramuscularly 50 µL of the rehydrate formulations of Example 1-1, Example 1-4, Example 1-8, and Example 1-12, and the model sample, respectively, for immunization: 14 days after primary immunization, secondary immunization was performed with an equal dose; 14 and 28 days after primary immunization, serum was collected and detected for SARS-CoV-2 specific IgG and neutralizing antibody response levels.

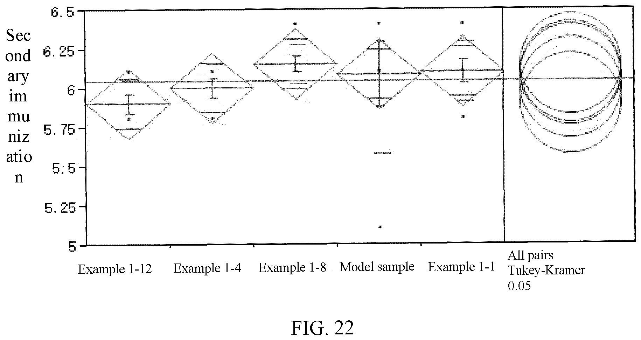

The immune effect on day 14 after primary immunization is shown in FIG. 21, and the immune effect on day 28 after primary immunization is shown in FIG. 22. It could be seen from the experimental results that Example 1-1, Example 1-4, Example 1-8, and Example 1-12 could all elicit sufficient *in vivo* immune response, and the immune response level of Example 1-8 on day 28 after primary immunization even exceeded that of the model sample, indicating that the freeze-drying process designed in the present disclosure can maintain or even improve the *in vivo* immune effect of mRNA-LNP.

## Claims

1. A nucleic acid-lipid nanoparticle formulation, comprising a freeze-drying protective agent comprising one or more of the following components: sucrose, trehalose, and mannitol.

2. The nucleic acid-lipid nanoparticle formulation according to claim 1, wherein a mass ratio of sucrose to trehalose to mannitol is 5-15:5-11:1-5.

3. A preparation method for the nucleic acid-lipid nanoparticle formulation according to claim 1, wherein sucrose, trehalose, and mannitol are dissolved in nuclease-free water according to corresponding proportions.

4. Use of a freeze-drying protective agent in the manufacture of a medicament, wherein the freeze-drying protective agent consists of the following components: sucrose and trehalose, wherein a mass ratio of sucrose to trehalose is 5-15:5-11.

5. The use according to claim 4, wherein the freeze-drying protective agent is used for nucleic acid-lipid nanoparticles, and preferably, the nucleic acid-lipid nanoparticle comprises ssDNA, dsDNA, mRNA, lncRNA, siRNA, saRNA, shRNA, ASO, plasmid, circRNA, circDNA, miRNA, CRISPR-Cas, ployI:C, SamRNA, or 5'-pppRNA.

6. Use of a freeze-drying protective agent in the manufacture of a medicament, wherein the freeze-drying protective agent comprises the following components: sucrose, trehalose, and mannitol, wherein a mass ratio of sucrose to trehalose to mannitol is 5-15:5-11:1-5, the freeze-drying protective agent is used for nucleic acid-lipid nanoparticles, and preferably, the nucleic acid-lipid nanoparticle comprises ssDNA, dsDNA, mRNA, lncRNA, siRNA, saRNA, shRNA, ASO, plasmid, circRNA, circDNA, miRNA, CRISPR-Cas, ployI:C, SamRNA, or 5'-pppRNA.

7. The use according to claim 5 or 6, wherein the nucleic acid-lipid nanoparticles are added into a solution of the freeze-drying protective agent for freeze-drying.

8. The use according to claim 7, wherein the freeze-drying comprises the following steps: pre-freezing, sublimation drying, and desorption drying.

9. The use according to claim 8, wherein the pre-freezing is performed at a temperature of -80°C to -50°C for a period of 2-6 h.

10. The use according to claim 8, wherein the sublimation drying is performed at a temperature of -60°C to 0°C with an end temperature of -5°C to 0°C and vacuum pressure controlled to be ≤ 10 Pa.

11. The use according to claim 8, wherein the desorption drying is performed at a temperature of 0-35°C with an end temperature of 30-35°C, running time of 2-8 h, and vacuum pressure controlled to be ≤ 10 Pa.

12. The use according to claim 11, wherein the sublimation drying is divided into 2-8 stages in a gradient heating mode, with each of the stages being in a heating gradient of 5-30°C and maintained for 0.5-10 h.

13. The use according to claim 8, wherein the medicament is a vaccine.

14. The use according to claim 13, wherein the vaccine is a vaccine for preventing cancer, viral infection, bacterial infection, and fungal infection.

15. The use according to claim 14, wherein the virus is selected from: norovirus, Ebola virus, coronavirus, cytomegalovirus, Dengue virus, Zika virus, enterovirus, hepatitis virus, herpes simplex virus, human papilloma virus, influenza virus, Marburg virus, measles virus, poliovirus, rabies virus, and rotavirus.

16. The use according to claim 14, wherein the virus is coxsackievirus.

17. The use according to claim 13, wherein the freeze-drying agent is added to the vaccine in an amount of 5-20 w/w%.

18. The use according to claim 4 or 5, wherein the medicament is an oral formulation, an intramuscular injectable formulation, an intravenous injectable formulation, or an inhalant formulation.

19. The use according to claim 18, wherein the inhalant formulation is an aerosolized inhalant formulation or a dry powder inhalant formulation.
